# EUROPÄISCHE PATENTSCHRIFT

(11) **EP 2 688 533 B1**
(45) Veröffentlichungstag und Bekanntmachung des Hinweises auf die Patenterteilung: **15.02.2017**
(21) Anmeldenummer: 12713912.9
(22) Anmeldetag: 21.03.2012
(51) Int. Cl.: A61F 13/00

(54) **PRIMÄRAUFLAGE FÜR DIE FEUCHTE WUNDVERSORGUNG UND VERFAHREN ZU DEREN HERSTELLUNG**
PRIMARY DRESSING FOR MOIST WOUND HEALING, AND METHOD FOR PRODUCING SAID PRIMARY DRESSING
PANSEMENT PRIMAIRE DESTINÉ À SOIGNER DES PLAIES EN MAINTENANT UN MILIEU HUMIDE, ET SON PROCÉDÉ DE FABRICATION

(30) Priorität: 21.03.2011 DE 102011005876; 08.07.2011 DE 102011051661
(43) Veröffentlichungstag der Anmeldung: 29.01.2014
(73) Patentinhaber: KET Kunststoff- und Elasttechnik GmbH Liegau-Augustusbad, 01454 Radeberg (DE)
(72) Erfinder: WENSKE, Günther, 01454 Radeberg (DE); BOETTCHER, Gunter, 01561 Würschnitz (DE)
(74) Vertreter: Kailuweit & Uhlemann Patentanwälte Partnerschaft mbB
(86) Internationale Anmeldenummer: PCT/EP2012/054953
(87) Internationale Veröffentlichungsnummer: WO 2012/126929

(56) Entgegenhaltungen:
- EP-A1- 0 596 215
- DE-A1- 4 407 031
- GB-A- 2 471 021
- US-A- 4 034 751
- US-A- 4 921 704

## Beschreibung

Die Erfindung betrifft eine Primärauflage für die feuchte Wundversorgung in Form eines Gitters oder Netzes, welches längliche Körper aus Silikon und ggf. darin eingebetteten Wirkstoffen und/oder Farbstoffen enthält, sowie Verfahren zu deren Herstellung.

Chronische Wunden, die sehr viel Exsudat bilden und bei denen eine Granulation gefördert werden soll, werden nach dem Prinzip der feuchten Wundversorgung behandelt. Dabei wird ein Wundverband auf die Wunde aufgebracht, der zur Aufnahme von Wundsekret geeignet ist, die Austrocknung der Wundoberfläche verhindert und so das physiologische Wundmilieu aufrechterhält. Der Heilungsprozess der Wunde ist mit Gefäß-, Fibroblasten- und Epithelwachstum verbunden, welche zur Ausbildung neuen Gewebes in die Wunde einwachsen. Ist die Wunde während des Heilungsprozesses mit einer feinfaserigen Wundauflage bedeckt, so besteht einerseits das Risiko von Infektionen durch Einwachsen des Gewebes in die Fasern des Wundverbands. Andererseits ist beim Einwachsen von Gewebe in den Wundverband ein atraumatisches Abziehen des Wundverbands nicht möglich und die Wundoberfläche wird bei Entfernung des Wundverbands zerstört und der Heilungsprozess dadurch verlängert.

Zur Förderung der Granulation kommen in der Wundbehandlung offenporige Schaumstoffe zum Einsatz, die am Wundgrund anhaften. Hydrophile Schaumstoffe sorgen durch eine verstärkte Kapillarwirkung für einen schnellen Transport des Exsudats nach außen und gewährleisten so ein ausgeglichenes feuchtes Wundmilieu. Aufgrund der Offenporigkeit besteht jedoch die Gefahr der Verwachsung mit der Wunde.

Funktionelle Wundverbände sind aus drei Elementen aufgebaut: Primärauflage, Sekundärauflage und Fixierung. Die Primärauflage liegt in der Verwendung direkt auf der Wunde auf und soll ein feuchtes Wundmilieu aufrechterhalten und überschüssiges Exsudat von der Wunde wegtransportierten. Die Primärauflage darf nicht mit der Wunde verkleben, so dass ein atraumatisches Abziehen des Wundverbands möglich ist. Die Sekundärauflage, die auf der von der Wunde abgewandten Seite der Primärauflage angeordnet ist, enthält den Saugkörper, beispielsweise einen offenporigen Schaumstoff, von dem das Wundexsudat aufgenommen wird. Gegebenenfalls enthält die Sekundärauflage noch eine Verteilschicht, die in der Anwendung zwischen Primärauflage und Saugkörper angeordnet ist und für eine gleichmäßige Verteilung des Wundexsudats sorgt. Um ein Durchnässen des Verbands nach außen zu verhindern, ist in der Sekundärauflage auf der von der Wunde abgewandten Seite gegebenenfalls eine flüssigkeitsundurchlässige aber atmungsaktive Schutzschicht angeordnet. Um die Position von Primär- und Sekundärauflage bei der Wundbehandlung zu fixieren, wird ein Verband angebracht. Geeignete Materialien für die Fixierung sind Fixierbinden, Schlauchverbände oder Fixierpflaster.

Die Zusammensetzung der direkt auf der Wunde aufliegenden Primärauflage ist für den Heilungsprozess von enormer Bedeutung. Zur Verhinderung des Verklebens der Primärauflage mit der Wundoberfläche werden poröse Primärauflagen mit einer hydrophoben Silikonoberfläche vorgeschlagen. Silikone sind biokompatibel und lösen keine Entzündungsreaktionen aus. Durch ihre hydrophobe Oberfläche können Zellen nur gering bis gar nicht an der Oberfläche des Silikons adhärieren. Sie verkleben nicht mit der Wunde und ermöglichen so ein atraumatisches Abziehen. Aufgrund ihrer guten Dampfdurchlässigkeit sind Silikone besonders atmungsaktiv. Durch die Poren kann das Wundexsudat gut abtransportiert werden.

DE 4407031 A1 offenbart ein silikonisiertes Faservlies, welches aus einem Trägermaterial aus Kunststofffasern, insbesondere Polyester oder Polypropylen, und einer darauf aufgebrachten ein- oder zweiseitigen Silikonbeschichtung besteht. Bei der Herstellung wird das Trägermaterial mit einem lösungsmittelfreien, additionsvernetzenden Silikon beschichtet. Dadurch wird auf dem Faservlies eine glatte Oberfläche erzeugt, die vorteilhaft nicht an der Wundoberfläche haften soll.

GB 2 471 021 A beschreibt elastische Netze, die aus Silikon hergestellt sein können. Die Netze können einerseits als Verpackungsmaterial in der Lebensmittelindustrie zum Einsatz kommen. Andererseits wird auch eine Anwendung zur Befestigung von Wundauflagen oder Bandagen auf einem Patienten vorgeschlagen. Es wird u.a. beschrieben, dass das Netz durch Stricken von Materialien wie Silikon erhalten werden kann. Dieses Netz kann zur Befestigung, in Kombination mit einer üblichen Wundauflage bzw. Bandage eingesetzt werden.

EP 0 595 215 A1 offenbart ein Material zur Aufbringung auf eine Wundoberfläche, welches einen Überzug mit einem Polymerfilm enthält, in den ein Chitinpulver eingebettet ist. Dazu werden u.a. das wasserlösliche Polymer und das Chitin dispergiert und ein Netz aus Polymeren in diese Dispersion getaucht. Das Netz wird dabei mit der Polymer-Chitin-Mischung benetzt.

In US 4 921 704 A wird eine Primärauflage offenbart, welche ein stabilisierendes elastisches Netz aufweist, das mit einer hydrophoben Gelschicht überzogen ist. Die Gelschicht kann bevorzugt eine Silikongelschicht oder Polyurethangelschicht sein. Das Netz dient einerseits zur Stabilisierung der Gelschicht. Andererseits ist es erforderlich, um ein Porensystem bereitzustellen, so dass das Wundexsudat durch die gebildeten Poren abtransportiert werden kann. Die Gelschicht kann eingebettete Wirkstoffe (z.B. antibakterielle Wirkstoffe) enthalten oder auch Substanzen, die die Wundheilung anregen.

DE 44 07 031 A1 beschreibt ein Faservlies, welches eine Silikonbeschichtung aufweist. Das Vlies enthält ein Trägermaterial aus Kunststofffasern (beispielsweise Polyester oder Polypropylen), wobei auf dem Trägermaterial eine ein- oder zweiseitige Silikonbeschichtung aufgebracht ist. Das Faservlies wird hergestellt, indem das Trägermaterial mit einem lösungsmittelfreien, additionsvernetzenden Silikon beschichtet wird. Es wird dadurch auf dem Vlies eine glatte Oberfläche erzeugt.

In US 4 034 751 A werden Polymerisatfolien offenbart, die als Wundauflagen eingesetzt werden. Die Folien sollen u.a. zur Abdeckung von Brandwunden zum Einsatz kommen.

WO 87/05206 offenbart eine Primärauflage, welche ein stabilisierendes elastisches Netz mit einer hydrophoben Gelschicht, insbesondere einer Silikongelschicht oder Polyurethangelschicht, umfasst. Das Netz ist dabei einerseits für die Stabilisierung erforderlich und dient andererseits zur Bereitstellung des Porensystems, welches für den Transport des Wundexsudats durch die Primärauflage notwendig ist. In die hydrophobe Gelschicht können antibakterielle Wirkstoffe oder Substanzen, die die Wundheilung anregen, eingeschlossen sein. Um Infektionen durch sich ablösende Fasern aus dem Netz zu vermeiden, ist es erforderlich, dass die hydrophobe Gelschicht das Netz vollständig (ohne Fehlstellen) umhüllt. Durch die hydrophobe Gelschicht wird einerseits die Fixierung an der Wundstelle sichergestellt, andererseits besteht gerade durch die Haftung der Primärauflage aufgrund des Gels das Risiko, dass die Wundstelle durch ein Abziehen der Primärauflage beschädigt wird.

Als Weiterentwicklung der WO 87/05206 offenbart EP 0633758 B1 zum Schutz vor Durchnässen einen Wundverband mit einer Primärauflage aus einem elastischen textilen Netz, welches analog zu WO 87/05206 mit einer hydrophoben Silikongelschicht beschichtet und umhüllt ist. Weiterhin umfasst die Wundauflage nach EP 0633758 B1 einen absorbierenden Körper (Saugkörper) und eine Flüssigkeitssperrschicht.

Wundauflagen nach DE 4407031 A1, WO 87/05206 und EP 0633758 B1 haben gemeinsam, dass sie eine poröse Stützstruktur enthalten, die mit einer Silikonbeschichtung versehen ist. Neben der aufwändigen Herstellung in zwei Schritten, bei der zunächst die zu beschichtende Stützstruktur bereitgestellt wird und diese anschließend mit Silikon oder Silikongel überzogen wird, haben derartige Wundauflagen den Nachteil, dass bei Fehlstellen der Umhüllung abgelöste Fasern der Stützstruktur in die Wunde eindringen können und Infektionen verursachen können. Durch das Einwachsen des Gewebes in die Fasern der Stützstruktur ist auch ein atraumatisches Entfernen der Wundauflage gefährdet. Die Erfindung macht sich daher zur Aufgabe, eine Primärauflage mit einem verringerten Entzündungsrisiko bereitzustellen und Verfahren zu deren Herstellung anzugeben.

Die Aufgabe wird erfindungsgemäß gelöst durch eine elastische Primärauflage für die feuchte Wundversorgung in Form eines Gitters oder Netzes aus elastischen länglichen Körpern, wobei das Gitter oder Netz nicht-resorbierbare längliche Körper enthält. Die nicht-resorbierbaren länglichen Körper bestehen aus Silikon und ggf. mindestens einem Zusatzstoff ausgewählt aus pharmakologischen Wirkstoffen, Kollagen, Hydrokolloiden und/oder Farbstoffen. Bevorzugt sind nicht-resorbierbare längliche Körper, die aus Silikon und ggf. Farbstoffen bestehen.

In bevorzugten Ausführungsformen der Erfindung enthält das Gitter oder Netz zusätzlich zu den nicht-resorbierbaren länglichen Körpern resorbierbare längliche Körper, welche mindestens eine resorbierbare und bioaktive organische Verbindung, insbesondere ausgewählt aus Kollagen und Hydrokolloiden, enthalten.

Die erfindungsgemäße Primärauflage stellt keine mit Silikon beschichtete offenporige Struktur dar, sondern das Material einzelner Stränge der Gitter- oder Netzstruktur selbst ist Silikon, welches ggf. Wirk- und/oder Farbstoffe enthält, die entweder in der Silikonmatrix enthalten sind oder als Bestandteil eines resorbierbaren Überzugs auf der Oberfläche des Gitters oder Netzes vorliegen.

Optional sind in der Gitter- oder Netzstruktur weitere Stränge aus resorbierbaren Materialien enthalten, die bei der Anwendung in der Wundversorgung abgebaut werden und dabei vorzugsweise bioaktive Substanzen und/oder Wirkstoffe freisetzen.

Silikon wird biologisch nicht abgebaut (es ist also nicht resorbierbar) und ist sehr reaktionsträge. Allergische Reaktionen bei oberflächlichen Anwendungen sind für Silikon nicht bekannt. Da mit der erfindungsgemäßen Primärauflage keine ummantelte Stützstruktur vorliegt, verursacht die erfindungsgemäße Primärauflage vorteilhaft keine Entzündungsreaktionen gegen weitere Bestandteile der Primärauflage.

Eine erfindungsgemäße Primärauflage liegt in Form eines flachen Gitters oder Netzes vor. Darin sind nicht-resorbierbare elastische längliche Körper aus Silikon, in welches ggf. pharmakologische Wirkstoffe, Kollagen, Hydrokolloide und/oder Farbstoffe eingebettet sind, zu einer offenporigen Struktur verknüpft. Durch die offenporige Gestaltung der Primärauflage ist sichergestellt, dass ein effektiver Abtransport des Exsudats möglich ist. Durch die Verwendung des inerten und bioverträglichen Silikons ist ein schmerz- und beschädigungsfreies Entfernen der Wundauflage möglich.

Neben der atraumatischen Entfernung ist die aktive Förderung der Wundheilung ein Aspekt von bevorzugten Ausgestaltungen der Primärauflage. Dies wird bewerkstelligt, indem bioaktive Substanzen und/oder pharmakologische Wirkstoffe in die Primärauflage eingebracht werden. Diese liegen vorzugsweise als Bestandteil der nicht-resorbierbaren länglichen Körper im Silikon eingebettet vor und/oder sind Teil eines resorbierbaren Überzugs auf der Oberfläche des Gitters oder Netzes und/oder es sind zusätzlich zu den nicht-resorbierbaren länglichen Körpern in der Primärauflage weitere resorbierbare längliche Körper enthalten, die resorbierbare und bioaktive organische Verbindungen enthalten. Auf diese Weise ist es möglich, in die Primärauflage bioaktive Substanzen oder Wirkstoffe einzubringen, die in der Anwendung auf der Wunde freigesetzt werden. In jedem Fall ist die Primärauflage so gestaltet, dass auch nach Resorption der resorbierbaren länglichen Körper ein nicht-resorbierbares Flächengebilde mit einer Gitter- oder Netzstruktur verbleibt.

Resorbierbare Materialien im Sinne der Erfindung unterscheiden sich von nicht-resorbierbaren Materialien darin, dass sie in der Anwendung auf der Wunde beispielsweise durch in der Wunde befindliche Zellen verstoffwechselt und dadurch mit der Zeit abgebaut werden. Nicht-resorbierbare Materialien werden nicht verstoffwechselt und bleiben dauerhaft erhalten. Bioaktive Substanzen im Sinne der Erfindung sind Substanzen, die die Wundheilung aktiv fördern. Bevorzugte bioaktive Substanzen sind Kollagene. Diese sind natürliche Bestandteile der Haut und beschleunigen den Heilungsprozess durch die Neubildung von Haut. Die erfindungsgemäße Primärauflage sorgt aufgrund ihrer offenporigen, flexiblen Struktur für ein ideales Wundmilieu und zusätzlich dazu wird durch Kollagen der Heilungsprozess gefördert.

Weiter bevorzugte resorbierbare Materialien sind Hydrokolloide (insbesondere Alginate). Bevorzugt liegen diese in Form eines Hydrogels vor.

Überzüge, die auf erfindungsgemäßen Primärauflagen vorliegen, sind ausschließlich resorbierbar. Es liegen keine nicht-resorbierbaren Überzüge auf erfindungsgemäßen Primärauflagen (insbesondere keine Überzüge, die Silikone enthalten) vor. Bei der Aufbringung von resorbierbaren Überzügen wird die Primärauflage vorzugsweise so lang auf der Wundfläche belassen, bis der Überzug abgebaut ist und eine atraumatische Entfernung der Primärauflage möglich ist.

Erfindungsgemäße Primärauflagen sind durch Verfahren des dreidimensionalen Druckens (3D printing), Siebdruck oder Textilverarbeitungsverfahren, insbesondere Weben, Wirken oder Stricken, erhältlich. In Abhängigkeit vom jeweiligen Herstellungsverfahren ist die Gitter- oder Netzstruktur der Primärauflage unterschiedlich fixiert. Ist die Primärauflage ein durch 3D Printing oder Siebdruck hergestelltes Gitter, so durchdringen sich die länglichen Körper an deren Kreuzungspunkten im Gitter, so dass eine Fixierung aufgrund der Durchdringung gewährleistet ist. Ist die Primärauflage ein durch Textilverarbeitungsverfahren hergestelltes Gitter oder Netz, so durchdringen sich die einzelnen Stränge an deren Kreuzungspunkten nicht. Die Fixierung der Gitter- oder Netzstruktur der Primärauflage wird in diesem Fall durch einen am Rand der Primärauflage aufgebrachten Kleberand gewährleistet. Grundsätzlich sind beliebige medizinisch zugelassene Kleber dazu geeignet (z.B. Hot-Melts). Bevorzugte Materialien für den Kleberand sind Klebsilikone, da sich diese hervorragend mit dem Silikon der Gitter- oder Netzstruktur verbinden.

Die dreidimensionale Struktur der erfindungsgemäßen Primärauflage ist offenporig und herstellungsbedingt ein Gitter oder Netz. Primärauflagen in Form eines Gitters sind durch 3D printing, Siebdruck oder Weben erhältlich. Primärauflagen in Form eines Netzes sind durch Wirken oder Stricken erhältlich. Die Struktur der Primärauflage ist offenporig, wodurch der Abtransport des Wundexsudats möglich ist.

Liegt die Primärauflage in Form eines Gitters vor, so umfasst dieses mindestens zwei gekreuzte Lagen von parallelen länglichen Körpern (Strängen). Bevorzugte Primärauflagen bestehen aus zwei gekreuzten Lagen paralleler Stränge. Für die Anwendung bei tiefen Wunden sind Primärauflagen mit mehr als zwei Lagen paralleler Stränge bevorzugt.

Die erfindungsgemäße Primärauflage ist elastisch und enthält nicht-resorbierbare längliche Körper, die nicht verstoffwechselt werden. Dies ermöglicht einerseits eine Anpassung an die Wundoberfläche und einen dauerhaften Schutz vor dem Verkleben der Wunde.

Silikone die in erfindungsgemäßen Primärauflagen als nicht-resorbierbare Bestandteile enthalten sind, sind vorzugsweise vernetzende Silikone, insbesondere elastische Silikonkautschuke. Silikonkautschuke enthalten als Grundpolymere Polyorganosiloxane, welche funktionelle Gruppen, insbesondere H-Atome, OH- und Vinylgruppen, enthalten, die für Vernetzungsreaktionen zugänglich sind.

Bevorzugt sind kaltvulkanisierende Silikonkautschuke (RTV), heißvulkanisierende Silikonkautschuke (HTV, LSR /Flüssigsilikon) und UV-vernetzende Silikone. Einkomponenten RTV-Silikonkautschuke polymerisieren unter dem Einfluss von Luftfeuchtigkeit, wobei die Vernetzung durch Kondensation von SiOH-Gruppen unter Bildung von Si,O-Bindungen erfolgt. Zweikomponenten RTV-Silikonkautschuke enthalten als weiteren Bestandteil einen Vernetzer, insbesondere Gemische aus Kieselsäuresestern oder organische Zinn-Verbindungen. Bei Vernetzung erfolgt die Bildung einer Si-O-Si-Brücke aus ≡Si-OR u. ≡Si-OH durch Alkohol-Abspaltung. HTV-Silikonkautschuke sind plastisch verformbare, fließfähige Silikone, die hochdisperse Kieselsäure und Vernetzungskatalysatoren, insbesondere organische Peroxide oder Platinverbindungen, enthalten. HTV-Silikonkautschuke vulkanisieren in der Regel durch Behandlung bei Temperaturen oberhalb von 100 °C.

Die Silikonoberfläche in einer erfindungsgemäßen Primärauflage weist vorzugsweise eine hydrophobe Oberfläche auf, die den Vorteil hat, dass Zellen nicht oder nur sehr schlecht auf der Oberfläche anwachsen können. Dennoch haften Primärauflagen mit einer hydrophoben Oberfläche gut an der Wundstelle, ohne diese zu verkleben. Durch Oberflächenmodifikation kann auf der erfindungsgemäßen Primärauflage eine hydrophile Oberfläche erzeugt werden. Dies fördert einen schnelleren Abtransport des Wundexsudats.

Bevorzugt sind erfindungsgemäße Primärauflagen mit einer hydrophoben Oberfläche, da diese aufgrund der geringen Zelladhärenz eine atraumatische Entfernung der Primärauflage begünstigt.

Vorzugsweise weisen die länglichen Körper der Primärauflage eine runde oder ovale Querschnittsfläche auf. Dadurch wird vorteilhaft die Auflagefläche der Primärauflage auf der Wunde minimiert. Auch dies trägt dazu bei, dass ein einfacheres Abziehen der Primärauflage von der Wunde ohne mechanische Zerstörung des neu gebildeten Gewebes möglich ist. Daher weist die erfindungsgemäße Primärauflage in Form eines Gitters oder Netzes mit runden oder ovalen länglichen Körpern eine hydrophile oder hydrophobe Oberfläche auf.

Abhängig von der Exsudatmenge der Wunde kann ein verbesserter Abtransport durch unterschiedliche Porengrößen erzielt werden. Im Sinne der Erfindung ist unter der Porengröße der Primärauflage der Abstand zweier nebeneinander angeordneter länglicher Körper (gemessen von der Außenseite der länglichen Körper) zu verstehen. Eine erfindungsgemäße Primärauflage weist vorzugsweise eine Porengröße von 0,5 - 1,2 mm auf. Bevorzugt ist eine Porengröße von 0,8 - 1 mm.

Die vertikale Ausdehnung der länglichen Körper der Primärauflage (für den Fall, dass diese eine runde Querschnittsfläche aufweisen, der Durchmesser der Querschnittsfläche der länglichen Körper) beträgt vorzugsweise 0,5 - 1 mm, besonders bevorzugt 0,6 - 0,8 mm.

Die vertikale Ausdehnung der gesamten Primärauflage (Dicke), welche mindestens zwei Lagen länglicher Körper umfasst, beträgt vorzugsweise maximal 4 mm, bevorzugt maximal 2 mm, besonders bevorzugt maximal 1,5 mm, ganz besonders bevorzugt zwischen 0,7 und 1,5 mm.

Bevorzugt wird die erfindungsgemäße Primärauflage in der für die Anwendung vorgesehenen Größe hergestellt, vorzugsweise mit einer Fläche von maximal 1600 cm², weiter bevorzugt maximal 400 cm² (wobei die Form der Primärauflage beliebig, z.B. rechteckig, quadratisch, rund, ellipsoid oder dergleichen sein kann). Ein Ausstanzen oder Zuschneiden der Primärauflagen, welches zu einem seitlich offenen und scharfkantigen Rand der Primärauflage führt (die eher zur Ansiedelung von Mikroorganismen neigen, als geschlossene Ränder), wird dadurch vermieden. Wird die Primärauflage in der Anwendungsgröße hergestellt, ist der seitliche Rand der Primärauflage bevorzugt schnittkantenfrei. Der seitliche Rand weist vorzugsweise eine geschlossene und abgerundete Struktur auf (atraumatische Randstruktur).

Weiter bevorzugt ist am seitlichen Rand der erfindungsgemäßen Primärauflage eine Abziehlasche aus nicht-resorbierbaren länglichen Körpern angebracht. Die nicht-resorbierbaren länglichen Körper bilden dabei die seitlich am Gitter oder Netz angeordnete Abziehlasche aus. Die Abziehlasche liegt in der Anwendung nicht unmittelbar auf der Wunde auf. Die Entfernung der Primärauflage von der Wunde ist dadurch erleichtert, da die Primärauflage durch ziehen an der Abziehlasche unkompliziert und atraumatisch entfernt werden kann.

Zur aktiven Förderung der Wundheilung enthält eine erfindungsgemäße Primärauflage vorzugsweise pharmakologische Wirkstoffe, vorzugsweise antibakterielle Wirkstoffe oder Wirkstoffe, die die Wundheilung fördern. Diese sind im Silikon enthalten, liegen in einem Überzug auf der Oberfläche der Gitter- oder Netzstruktur vor und/oder sind in resorbierbare längliche Körper der Gitterstruktur eingebettet.

Bevorzugt enthalten die resorbierbaren länglichen Körper einen antibakteriellen Wirkstoff, vorzugsweise ein Wundantiseptikum, besonders bevorzugt ein gegen Bakterien und Pilze wirksames Wundantiseptikum. Ein besonders bevorzugtes Wundantiseptikum ist Polihexanid.

Der antibakterielle Wirkstoff ist bevorzugt in einer antiseptisch wirksamen Konzentration in den resorbierbaren länglichen Körpern enthalten. Geeignete Konzentrationen für Polihexanid sind 0,01 bis 0,1 Vol.-%.

Vorzugsweise sind keine Farbstoffe in einer erfindungsgemäßen Primärauflage enthalten. Dadurch ist die Wundfläche bei Verwendung transparenter Silikone bereits ohne Ablösen der Primärauflage einsehbar. Gegebenenfalls sind medizinisch zugelassene und unbedenkliche Farbstoffe in das Silikon eingebettet.

Die Erfindung umfasst auch eine Wundauflage für die feuchte Wundversorgung, die eine erfindungsgemäße Primärauflage und eine Sekundärauflage mit einem porösen Saugkörper umfasst. Die Primärauflage liegt bei Verwendung unmittelbar auf der Wunde auf, die Sekundärauflage befindet sich auf der Seite von der Wundauflage, die bei Verwendung von der Wundfläche abgewandt ist. Vorzugsweise weist der poröse Saugkörper eine hydrophile Oberfläche auf. Bevorzugte poröse Saugkörper sind Schäume aus Polyurethan oder Silikon. Die Rohdichte des Schaumes beträgt vorzugsweise 100 bis 250 kg/m³, besonders bevorzugt 120 bis 180 kg/m³, bei einer bevorzugten Zellenzahl pro Laufzentimeter: 10 +/- 4 (Poren pro cm). Die Stauchhärte (also der physikalisch auf eine Fläche in Quadratmetern wirkende Druck in Pascal, der notwendig ist, um den Schaum um 40% zusammenzudrücken) des porösen Saugkörpers beträgt vorzugsweise 3-15 kPa, besonders bevorzugt 4 bis 8 kPa. Die Zugfestigkeit des porösen Saugkörpers beträgt vorzugsweise mindestens 100 kPa (bestimmt nach DIN 53571A). Die Bruchdehnung des porösen Saugkörpers beträgt vorzugsweise mindestens 60 % (bestimmt nach DIN 53571 A). Bevorzugt hat der poröse Saugkörper eine vertikale Ausdehnung (Dicke) von 5 bis 25 mm.

Die Sekundärauflage umfasst neben dem porösen Saugkörper vorzugsweise eine Flüssigkeits-Sperrschicht und/oder eine geruchsbindende Schicht.

Die erfindungsgemäße Wundauflage enthält neben der Primär- und Sekundärauflage vorzugsweise eine Fixierung. Die Fixierung ist bevorzugt ausgewählt aus Fixierbinden, Schlauchverbänden oder Fixierpflastern. Dadurch wird sichergestellt, dass die Position der Wundauflage bei der Wundbehandlung unverändert bleibt.

Erfindungsgemäße Primärauflagen sind durch verschiedene Verfahren herstellbar. Verfahren zur Herstellung erfindungsgemäßer Primärauflagen durch 3D Printing, Siebdruck und Weben, Wirken oder Stricken sind ebenfalls Gegenstand der Erfindung. Die erfindungsgemäße Primärauflage wird bevorzugt durch Verfahren des 3D-Printing hergestellt, bei dem eine schnell aushärtende viskose Masse Schicht für Schicht durch Spritzen in Form gebracht wird. Bei diesem Verfahren werden auf dem Computer vorhandene Daten direkt zur Herstellung eines dreidimensionalen Körpers umgesetzt.

Im erfindungsgemäßen Verfahren zur schichtweisen Herstellung einer Primärauflage mit einer Gitterstruktur aus mindestens zwei Schichten sich kreuzender länglicher Körper mittels 3D-Printing wird zur Bereitstellung der Gitterstruktur eine erste Schicht aus zueinander parallel angeordneten länglichen Körpern aufgetragen und anschließend auf die erste Schicht eine zweite Schicht aus zueinander parallel angeordneten länglichen Körpern aufgetragen, wobei die länglichen Körper der ersten Schicht und die länglichen Körper der zweiten Schicht zueinander in einem Winkel von 1 bis 90 ° versetzt angeordnet sind, so dass sich die länglichen Körper der ersten und der zweiten Schicht kreuzen.

Die Auftragung der länglichen Körper erfolgt durch Extrusion aus einer viskosen Masse, wobei die Masse aus vernetzendem Silikon und ggf. pharmakologischen Wirkstoffen und/oder Farbstoffen besteht. Nach Vernetzung des Silikons ist ein elastischer Feststoff erhältlich. Verfahren des Extrudierens sind aus dem Stand der Technik bekannt. Bevorzugt erfolgt die Extrusion durch eine Düse mit einem maximalen Ausdehnung (bei rundem Querschnitt: maximalen Durchmesser) von 0,5 - 1 mm (hierin auch "Mikroextrusion").

Die Herstellung der Primärauflage mittels 3D Printing erfolgt schichtweise, wobei zunächst eine erste Schicht zueinander parallel angeordneter länglicher Körper aufgetragen wird und anschließend auf die erste Schicht eine zweite Schicht aus zueinander parallel angeordneten länglichen Körpern aufgetragen wird. Ggf. werden auf dieselbe Weise weitere Schichten länglicher Körper aufgetragen. Dabei sind die länglichen Körper unterschiedlicher Schichten zueinander in einem Winkel von 1 bis 90° versetzt angeordnet, so dass sich die länglichen Körper der aufeinanderliegenden Schichten kreuzen. Es werden mindestens zwei Schichten länglicher Körper im erfindungsgemäßen Verfahren aufgetragen. Besonders bevorzugt werden genau zwei Schichten länglicher Körper aufgetragen, so dass eine Gitterstruktur mit zwei Lagen sich kreuzender länglicher Körper erhältlich ist. Die länglichen Körper, die im erfindungsgemäßen Verfahren zur Bereitstellung der Gitterstruktur aufgetragen werden, weisen vorzugweise eine runde oder ovale Querschnittsfläche auf.

Pharmakologische Wirkstoffe, vorzugsweise antibakterielle Wirkstoffe und/oder Wirkstoffe, die die Wundheilung fördern, sind gegebenenfalls in der viskosen Masse, die durch Extrusion (insbesondere Mikroextrusion) im vorgenannten Herstellungsverfahren verarbeitet wird, enthalten. Weiterhin sind gegebenenfalls Farbstoffe, insbesondere mineralische Pigmentpräparationen, welche Pigmente in Kombination mit einem Silikonöl enthalten, in der viskosen Masse enthalten.

Das Verfahren zur Herstellung einer erfindungsgemäßen Primärauflage mittels 3D Printing bietet die Möglichkeit, neben den nicht-resorbierbaren Strängen aus Silikon (und ggf. Wirkstoffen und/oder Farbstoffen) auch resorbierbare Stränge in die Gitterstruktur einzubauen. Dafür werden längliche Körper in derselben Weise wie für die länglichen Körper aus Silikon und ggf. Wirkstoffen und/oder Farbstoffen beschrieben aus einer viskosen Masse geformt und in das Gitter eingearbeitet. Die viskose Masse zur Bereitstellung der resorbierbaren Stränge enthält dabei ausschließlich resorbierbare Materialien, insbesondere mindestens eine resorbierbare und bioaktive organische Verbindung, besonders bevorzugt ausgewählt aus Kollagen, Hydrokolloiden und pharmakologischen Wirkstoffen. Besonders bevorzugt davon ist Kollagen.

Im erfindungsgemäßen Verfahren zur Herstellung einer Primärauflage mit einer Gitterstruktur mittels Siebdruck wird die Gitterstruktur durch Siebdruck aus einer viskosen Masse aus vernetzendem Silikon und ggf. pharmakologischen Wirkstoffen und/oder Farbstoffen erzeugt. Es wird dabei ein Gitter aus ggf. mehreren Lagen erzeugt, wobei nach jeder aufgetragenen Lage die Vernetzung des Silikons durchgeführt wird.

Für die Verfahren zur Herstellung einer Primärauflage mittels 3D Printing oder Siebdruck weist die viskose Masse, die aus Silikon mit ggf. pharmakologischen Wirkstoffen und/oder Farbstoffen besteht, und/oder die viskose Masse zur Bereitstellung der resorbierbaren Stränge vorzugsweise eine Viskosität von 300.000 mPas bis 1.000.000 mPas bei einer Scherrate von 10 s⁻¹ auf (bestimmt im Rotationsviskosimeter).

Zur Herstellung einer erfindungsgemäßen Primärauflage mittels 3D Printing oder Siebdruck sind vernetzende elastische Silikonkautschuke, insbesondere LSR und RTV-Silikonkautschuk, Flüssigsilikon, HTV-Silikonkautschuk oder UV-vernetzendes Silikon, bevorzugt.

Je nach Art des eingesetzten Silikons erfolgt die Vernetzung des Silikons durch unterschiedliche Reaktionen. Einkomponenten RTV-Silikonkautschuke vernetzen, ohne dass weitere Verfahrensschritte erforderlich sind, durch Luftfeuchtigkeit. Zweikomponenten RTV-Silikonkautschuke werden vor der Verarbeitung der viskosen Masse in erfindungsgemäßen Verfahren (3D Printing oder Siebdruck) angemischt, wodurch die Vernetzung gestartet wird. Nach der Herstellung der Primärauflage sind somit keine weiteren Verfahrensschritte notwendig, um die Aushärtung der viskosen Masse zu bewirken. Primärauflagen auf Basis von HTV-Silikonkautschuk werden nach der Verarbeitung der viskosen Masse zur Gitterstruktur durch Temperaturbehandlung bei Temperaturen oberhalb von 100 °C vernetzt. Primärauflagen auf Basis von UV-vernetzbarem Silikon werden nach der Bereitstellung der Gitterstruktur UV-Strahlung ausgesetzt, wodurch das Silikon vernetzt wird.

Die Erfindung umfasst auch ein Verfahren zur Herstellung einer elastischen Primärauflage für die feuchte Wundversorgung in Form eines Gitters oder Netzes, bei dem das Gitter oder Netz durch Textilverarbeitung, insbesondere durch Weben, Wirken, Sticken oder Stricken, aus länglichen Körpern erzeugt wird, wobei die elastischen länglichen Körper aus Silikon und ggf. pharmakologischen Wirkstoffen und/oder Farbstoffen bestehen. Die länglichen Körper, vorzugsweise elastische Stränge aus Silikon und ggf. pharmakologischen Wirkstoffen und/oder Farbstoffen mit einer Dicke von maximal 1,5 mm, vorzugsweise maximal 1 mm, weisen bevorzugt eine runde oder ovale Querschnittsfläche auf. Bevorzugt werden resorbierbare längliche Körper (wie oben definiert) zusätzlich in das Gitter oder Netz eingearbeitet.

Durch die vorgenannten erfindungsgemäßen Verfahren werden bevorzugt Primärauflagen in der Anwendungsgröße (keine Bahnenware oder Großplatten) erzeugt. Bevorzugt werden daher durch erfindungsgemäße Verfahren Primärauflagen erzeugt, deren Fläche 1600 cm², vorzugsweise 400 cm², nicht überschreitet. Bevorzugt wird die Primärauflage in einem erfindungsgemäßen Verfahren nach der Bereitstellung des Gitters oder Netzes nicht durch Schneidverfahren (z.B. Stanzen oder Schneiden) weiterbehandelt. Dies führt vorteilhaft dazu, dass Primärauflagen mit einer schnittkantenfreien, vorzugsweise abgerundeten, Randstruktur erzeugt werden.

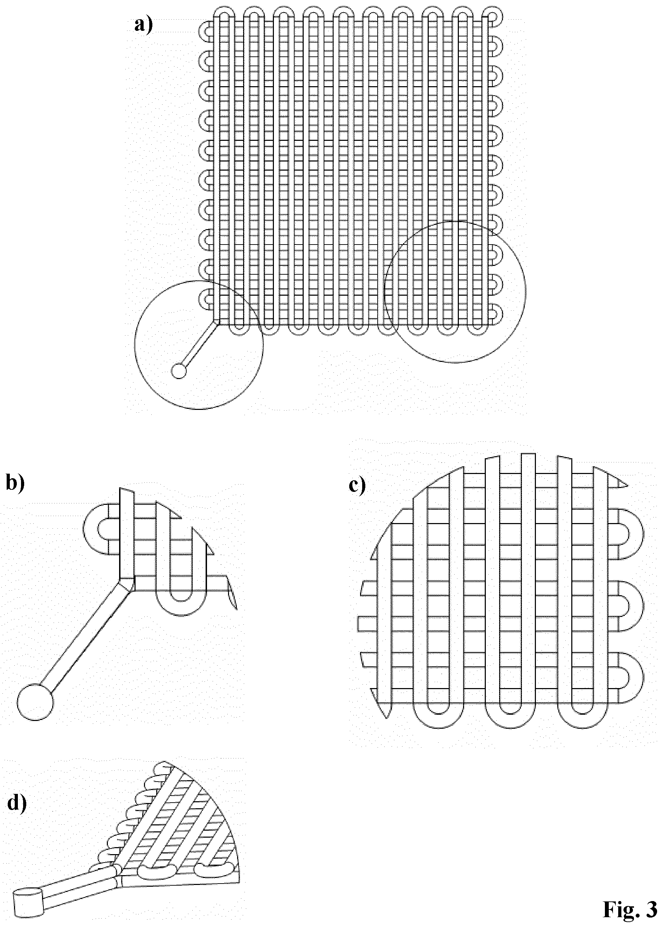

Für die Erzeugung einer derartigen Randstruktur sind Extrusionsverfahren, insbesondere das oben beschriebene 3D-Printing bevorzugt. Dabei wird die Gitterstruktur erzeugt, in dem die zueinander parallel angeordneten länglichen Körper aus einem zusammenhängenden Einzelstrang bestehen, der mäanderförmig angeordnet ist (siehe Fig. 3a, 3c). Dies hat den Vorteil, dass scharfkantige Ränder an der Primärauflage vermieden werden und auf diese Weise eine bessere Bioverträglichkeit der Primärauflage erzielt wird.

Vorzugsweise wird in einem der erfindungsgemäßen Verfahren an einem seitlichen Ende des Gitters oder Netzes eine Abziehlasche aus nicht-resorbierbaren länglichen Körpern erzeugt. Die Abziehlasche ist länglich ausgebildet, und ermöglicht vorteilhaft ein erleichtertes Ablösen der Primärauflage. Bevorzugt wird bei Extrusionsverfahren, insbesondere bei Verfahren des 3D-Printing, eine solche Abziehlasche erzeugt (beispielhaft siehe Fig. 3b, 3d).

Für den Fall, dass mit einem erfindungsgemäßen Verfahren resorbierbare Stränge in das Gitter oder Netz eingearbeitet werden, enthalten die resorbierbaren länglichen Körper bevorzugt einen antibakteriellen Wirkstoff, vorzugsweise ein Wundantiseptikum. Besonders bevorzugt sind Wundantiseptika, die sowohl gegen Bakterien als auch gegen Pilze wirksam sind. Ein ganz besonders bevorzugter antibakterieller Wirkstoff ist Polihexanid.

Nach Bereitstellung der Primärauflage durch die vorgenannten Verfahren und ggf. der Vernetzung des Silikons ist eine elastische Primärauflage für die feuchte Wundversorgung erhältlich, die eine hydrophobe Oberfläche aufweist.

In einem weiteren Verfahrensschritt wird die Oberfläche der durch das erfindungsgemäße Verfahren erhältlichen Primärauflage gegebenenfalls in eine hydrophile Oberfläche umgewandelt. Dies erfolgt bevorzugt durch Plasmabehandlung, Beflammung oder durch nasschemische Behandlung in einer Lösung mit MSA-Copolymeren, vorzugsweise durch Plasmabehandlung oder Beflammung.

Im Anschluss an eine Hydrophilierung wird auf die Oberfläche des Gitters oder Netzes vorzugsweise ein resorbierbarer Überzug aufgebracht, welcher mindestens einen pharmakologischen Wirkstoff und/oder mindestens eine resorbierbare organische Verbindung, insbesondere ausgewählt aus Kollagen und Hydrokolloiden, enthält. Bevorzugt sind wirkstoffhaltige und/oder kollagenhaltige Überzüge. Dieser Überzug wird in der Anwendung der Primärauflage auf der Wunde abgebaut und die Wirkstoffe und/oder bioaktiven Bestandteile werden freigesetzt.

Verfahren des 3D Printing, der Extrusion und Siebdruck sind an sich aus dem Stand der Technik bekannt. Die erfindungsgemäße Lösung bietet jedoch die Möglichkeit, diese Verfahren auch in der feuchten Wundversorgung einzusetzen. Daher umfasst die Erfindung auch die Verwendung von Verfahren des 3D Printing, der Extrusion und Siebdruck, insbesondere von Verfahren des 3D-Printing, zur Herstellung von Primärauflagen für die feuchte Wundversorgung. Besonders bevorzugt ist die Verwendung des 3D Printing, der Extrusion und Siebdruck von elastischen Silikonkautschuken zur Herstellung von Primärauflagen für die feuchte Wundversorgung.

Längliche Körper, insbesondere Fasern, aus Silikon sind aus dem Stand der Technik bekannt. Mit der Erfindung werden diese nunmehr vorteilhaft in der feuchten Wundversorgung eingesetzt. Daher umfasst die Erfindung auch die Verwendung von länglichen Körpern, insbesondere Fasern, aus Silikon und ggf. pharmakologischen Wirkstoffen und/oder Farbstoffen zur Herstellung von Primärauflagen für die feuchte Wundversorgung. Besonders bevorzugt ist dabei die Verwendung in einem der erfindungsgemäßen Verfahren, insbesondere Weben, Wirken, Sticken oder Stricken.

Gitter oder Netze, die durch 3D Printing, Siebdruck oder textilverarbeitende Verfahren erhältlich sind, sind aus dem Stand der Technik bekannt. In der Erfindung werden Gitter oder Netze mit länglichen Körpern aus Silikon, welche ggf. Wirkstoffe und Farbstoffe enthalten, für die feuchte Wundversorgung eingesetzt. Daher umfasst die Erfindung auch die Verwendung eines Gitter oder Netzes welches nicht-resorbierbare längliche Körper aus Silikon und ggf. mindestens einem Zusatzstoff ausgewählt aus pharmakologischen Wirkstoffen, Kollagen, Hydrokolloiden und/oder Farbstoffen umfasst und wobei das Gitter oder Netz durch 3D-Printing, Siebdruck, Wirken, Weben, Sticken oder Stricken, erhältlich ist, für die feuchte Wundversorgung, insbesondere als Primärauflage für die feuchte Wundversorgung.

Medizinische Indikationen, bei denen die erfindungsgemäße Lösung eingesetzt wird umfassen alle Wunden mit Exsudatbildung, insbesondere Ulzerationen in der Phase der Granulation. Besonders bevorzugte medizinische Indikationen sind chronische Unterschenkelgeschwüre, Spalthautentnahmestellen und Verbrennungen.

Die Erfindung bietet Verfahren, mit dem eine Primärauflage aus Silikon mit einem definierten Porensystem reproduzierbar herstellbar ist. Die dabei erhältliche Primärauflage für die feuchte Wundversorgung enthält nicht-resorbierbare längliche Körper aus Silikon und ggf. Wirkstoffen und/oder Farbstoffen. Eine zweistufige Beschichtung von stützenden Netzstrukturen aus Kunststoff mit Silikongelen ist im erfindungsgemäßen Verfahren nicht notwendig. Eine Gefahr von Entzündungen durch Fehlstellen in der Silikonschicht und der Unverträglichkeit des Körpers mit Bestandteilen der Stützstruktur ist daher vorteilhaft nicht gegeben.

Ansonsten weisen die erfindungsgemäßen Primärauflagen dieselben Vorteile, wie bekannte Wundauflagen mit Silikonbeschichtung auf. Durch die poröse, offenporige Struktur ist das Abführen des Wundexsudats sichergestellt. Die Silikonoberfläche erlaubt aufgrund der geringen Zelladhärenz ein atraumatisches Entfernen der Wundauflage. Durch den Einsatz runder oder ovaler Stränge in den Gitter- oder Netzstrukturen kann vorteilhaft die Auflagefläche verringert werden, was das Ablösen der Primärauflage weiter erleichtert.

Erfindungsgemäße Primärauflagen bieten den Vorteil, dass sie je nach den spezifischen Bedingungen des jeweiligen Patienten individuell ausgewählt werden können und eine geeignete Porengröße und/oder geeignete Strangquerschnitte ausgewählt werden können. Es ist eine effektive Exsudatableitung aufgrund der offenporigen Struktur möglich. Aufgrund der Kapillarwirkung ist ein schneller Abtransport von Wundexsudat gewährleistet und dennoch wird ein feuchtes Wundmilieu aufrecht erhalten. Aufgrund der Oberflächenspannung des Silikons haften erfindungsgemäße Primärauflagen auf der Wunde und benötigen nicht notwendigerweise weitere Kleber zur Fixierung.

Anhand folgender Figuren und Ausführungsbeispiele soll die Erfindung näher erläutert werden, ohne die Erfindung auf diese zu beschränken.
Fig. 1 Erfindungsgemäße Primärauflage aus zwei Schichten länglicher Körper mit runder Querschnittsfläche (Draufsicht), Angaben in [mm]. a) zeigt die Draufsicht der kompletten Primärauflage, b) zeigt eine Vergrößerung des in a) markierten Ausschnitts.
Fig. 2 Erfindungsgemäße Primärauflage aus zwei Schichten länglicher Körper mit runder Querschnittsfläche (Seitenansicht), Angaben in [mm]. a) zeigt die Seitenansicht der kompletten Primärauflage, b) zeigt eine Vergrößerung des in a) markierten Ausschnitts.
Fig. 3 Erfindungsgemäße Primärauflage aus zwei Schichten länglicher Körper mit schnittkantenfreier Randstruktur und Abziehlasche. a) zeigt die Draufsicht der kompletten Primärauflage, b) zeigt eine Vergrößerung des in a) links unten markierten Ausschnitts, c) zeigt eine Vergrößerung des in a) rechts unten markierten Ausschnitts, d) zeigt eine durch 3D Printing erzeugte Abziehlasche.

### Ausführungsbeispiel 1: Erfindungsgemäße Primärauflage aus zwei Schichten

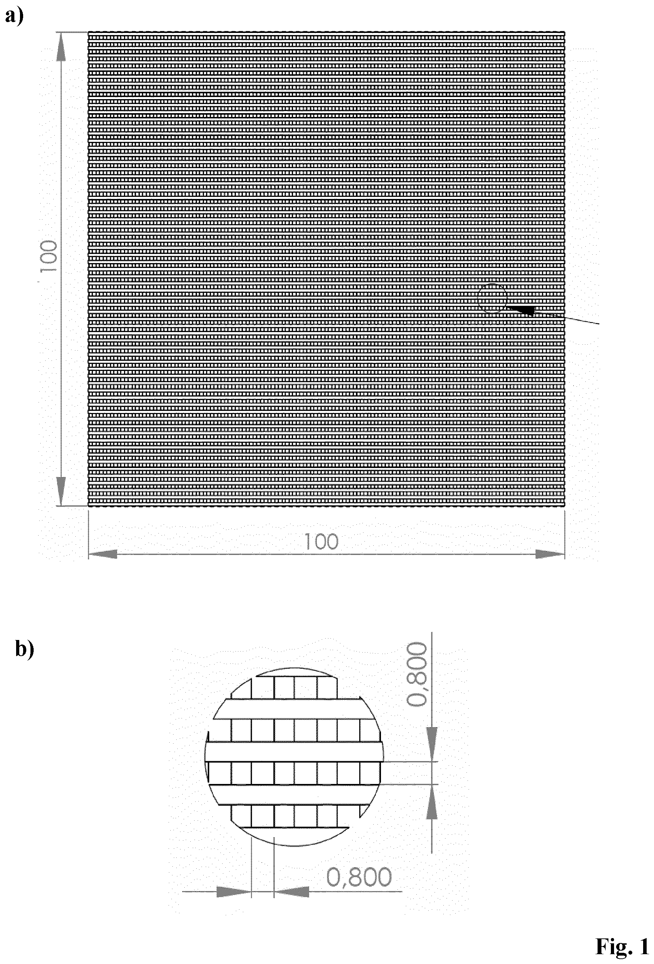

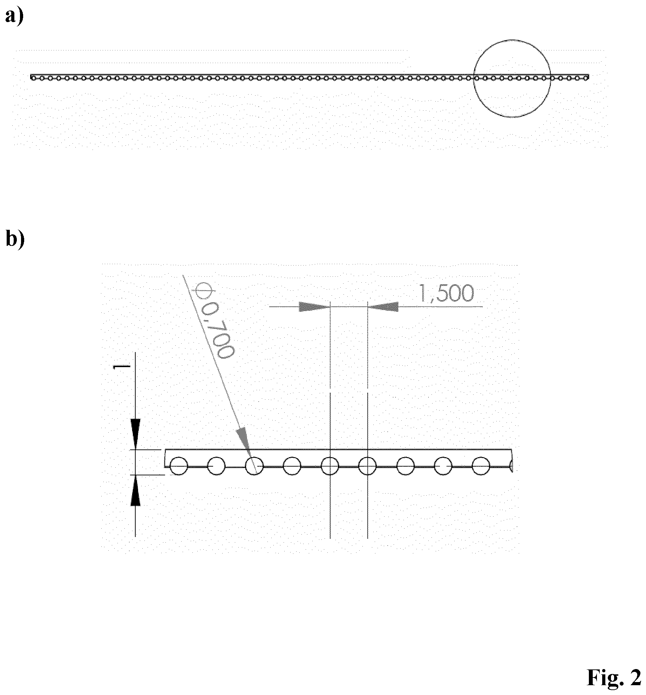

Fig. 1 und 2 zeigen jeweils Drauf- und Seitenansicht einer erfindungsgemäßen Primärauflage aus zwei Schichten parallel angeordneter runder Körper, die durch 3D-Printing von Flüssigsilikon erhalten wurde. Der Durchmesser der Querschnittsfläche der länglichen Körper beträgt 0,7 mm (Fig. 2b). Die Körper sind in einem Abstand von 0,8 mm angeordnet (**Fig. 1b**, entspricht 1,5 mm Abstand der Mittelpunkte der länglichen Körper, **Fig. 2b**). Die vertikale Ausdehnung der erfindungsgemäßen Primärauflage beträgt 1 mm (**Fig. 2b**). Die Primärauflage ist in einer quadratischen Form mit einer Seitenlänge von 100 mm bereitgestellt (**Fig. 1a**).

### Ausführungsbeispiel 2: Anwendung einer erfindungsgemäßen Primärauflage

Die erfindungsgemäße Primärauflage aus Ausführungsbeispiel 1 wurde so auf die Wundoberfläche gelegt, dass die Primärauflage den Wundrand um mindestens 1 cm überragte. Über die erfindungsgemäße Primärauflage wurde ein Polyurethanschaum derselben Größe angeordnet, der die Funktion eines porösen Saugkörpers erfüllt. Auf den Polyurethanschaum wurden sterile ES-Kompressen aufgebracht und die Wundauflage wurde mit einer Mullbinde fixiert.

Es wurde festgestellt, dass die Wundauflage mit der erfindungsgemäßen Primärauflage eine Austrocknung der Wunde verhindert und die Wundheilung unterstützt. Das gebildete Gewebe verwuchs nicht mit der Primärauflage. Die Wundauflage konnte ohne mechanische Zerstörung des gebildeten Gewebes entfernt werden. Entzündungsreaktionen und allergische Reaktionen aufgrund der Primärauflage wurden nicht festgestellt.

### Ausführungsbeispiel 3: Zweischichtige Primärauflage mit schnittkantenfreier Randstruktur und Abziehlasche

Fig. 3 zeigt eine Primärauflage in Gitterstruktur aus zwei Schichten paralleler länglicher Körper. Die Primärauflage ist durch Extrusion mittels 3D Printing erhältlich. Dafür wird die Gitterstruktur aus einem Einzelstrang einer viskosen Silikonmasse aufgetragen. Es wird zum Aufbau der Abziehlasche zunächst ein Teilstrang erzeugt, der einen unteren Teil der Abziehlasche ausbildet (siehe Fig. 3D). Um die Gitterstruktur zu erzeugen wird eine erste Schicht mäanderförmig angeordneter zueinander paralleler länglicher Körper erzeugt und anschließend aus demselben Einzelstrang eine weitere Schicht auf der ersten Schicht aufgebaut. Nach Fertigstellung der zweiten Schicht wird das Ende des Einzelstrangs auf der Oberfläche oder unmittelbar neben dem Teilstrang abgelegt. Diese bilden die Abziehlasche (Fig. 3b und d), die in der Anwendung nicht unmittelbar auf der Wunde aufliegt, so dass diese nicht mit der Wunde verklebt. Die Entfernung der Wundauflage ist dadurch erleichtert. Durch die mäanderförmige Anordnung der parallelen länglichen Körper wird eine abgerundete Randstruktur erzeugt. Es ist kein Zuschnitt der Primärauflage erforderlich, so dass die Randstruktur schnittkantenfrei verbleibt.

## Patentansprüche

1. Elastische Primärauflage für die feuchte Wundversorgung in Form eines Gitters oder Netzes aus elastischen länglichen Körpern, wobei das Gitter oder Netz nicht-resorbierbare längliche Körper enthält, wobei die nicht-resorbierbaren länglichen Körper aus Silikon und mindestens einem Zusatzstoff ausgewählt aus pharmakologischen Wirkstoffen, Kollagen, Hydrokolloiden und/oder Farbstoffen bestehen.

2. Primärauflage nach Anspruch 1, wobei das Gitter oder Netz zusätzlich resorbierbare längliche Körper umfassend mindestens eine resorbierbare und bioaktive organische Verbindung, ausgewählt aus Kollagen, Hydrokolloiden und pharmakologischen Wirkstoffen, enthält.

3. Primärauflage nach Anspruch 1 oder 2, wobei das Silikon vernetzendes Silikon ist, ausgewählt aus Flüssigsilikon, RTV-Silikonkautschuk, HTV-Silikonkautschuk und UV-vernetzbarem Silikon.

4. Primärauflage nach einem der vorhergehenden Ansprüche, wobei auf der Oberfläche des Gitters oder Netzes ein resorbierbarer Überzug vorliegt, welcher mindestens einen pharmakologischen Wirkstoff und/oder mindestens eine resorbierbare organische Verbindung, ausgewählt aus Kollagen und Hydrokolloiden, enthält.

5. Primärauflage nach einem der vorhergehenden Ansprüche, wobei antibakterielle Wirkstoffe und/oder Wirkstoffe zur Förderung der Wundheilung in das Silikon der nicht-resorbierbaren länglichen Körper eingebettet sind und/oder in einem resorbierbaren Überzug auf der Oberfläche des Gitters oder Netzes vorliegen.

6. Primärauflage nach einem der vorherigen Ansprüche, bei der die nicht-resorbierbaren länglichen Körper eine an dem Gitter oder Netz seitlich angeordnete Abziehlasche ausbilden.

7. Wundauflage für die feuchte Wundversorgung, umfassend
- eine Primärauflage nach einem der Ansprüche 1 bis 6, die bei Verwendung auf der Wundfläche aufliegt und
- eine Sekundärauflage, die auf der Seite von der Wundauflage angeordnet ist, die bei Verwendung von der Wundfläche abgewandt ist, wobei die Sekundärauflage einen porösen Saugkörper enthält.

8. Verfahren zur schichtweisen Herstellung einer elastischen Primärauflage für die feuchte Wundversorgung in Form eines Gitters aus mindestens zwei Schichten sich kreuzender länglicher Körper, wobei zur Bereitstellung des Gitters eine erste Schicht aus zueinander parallel angeordneten länglichen Körpern aufgetragen und anschließend auf die erste Schicht eine zweite Schicht aus zueinander parallel angeordneten länglichen Körpern aufgetragen wird, wobei die länglichen Körper der ersten Schicht und die länglichen Körper der zweiten Schicht zueinander in einem Winkel von 1 bis 90 ° versetzt angeordnet sind, so dass sich die länglichen Körper der ersten und der zweiten Schicht kreuzen, wobei die länglichen Körper durch Extrusion aus einer viskosen Masse erzeugt werden, wobei die Masse aus vernetzendem Silikon und pharmakologischen Wirkstoffen und/oder Farbstoffen besteht, und wobei nach Vernetzung des Silikons ein elastischer Feststoff erhältlich ist.

9. Verfahren zur Herstellung einer elastischen Primärauflage für die feuchte Wundversorgung in Form eines Gitters, bei dem das Gitter durch Siebdruck aus einer viskosen Masse erzeugt wird, wobei die Masse aus vernetzendem Silikon und ggf. pharmakologischen Wirkstoffen und/oder Farbstoffen besteht und wobei die Primärauflage in der Anwendungsgröße hergestellt wird.

10. Verfahren nach einem der Ansprüche 8 oder 9, **dadurch gekennzeichnet, dass** die viskose Masse aus vernetzendem Silikon und pharmakologischen Wirkstoffen und/oder Farbstoffen eine Viskosität von 300.000 mPas bis 1.000.000 mPas bei einer Scherrate von 10 s⁻¹ aufweist.

11. Verfahren zur Herstellung einer elastischen Primärauflage nach einem der Ansprüche 1 bis 6 für die feuchte Wundversorgung in Form eines Gitters oder Netzes, bei dem das Gitter oder Netz durch Weben, Wirken, Sticken oder Stricken von elastischen länglichen Körpern erzeugt wird, wobei die elastischen länglichen Körper aus Silikon und pharmakologischen Wirkstoffen und/oder Farbstoffen bestehen.

12. Verfahren nach einem der Ansprüche 9 bis 11, wobei an einem seitlichen Ende des Gitters oder Netzes eine Abziehlasche aus nicht-resorbierbaren länglichen Körpern erzeugt wird.

13. Verfahren nach einem der Ansprüche 8 bis 12, bei dem ein resorbierbarer Überzug, welcher mindestens einen pharmakologischen Wirkstoff und/oder mindestens eine resorbierbare organische Verbindung, ausgewählt aus Kollagen und Hydrokolloiden, enthält, auf die Oberfläche des Gitters oder Netzes aufgebracht wird.

14. Verwendung eines Gitter oder Netzes, welches nicht-resorbierbare längliche Körper enthält, wobei die nicht-resorbierbaren länglichen Körper aus Silikon und mindestens einem Zusatzstoff ausgewählt aus pharmakologischen Wirkstoffen, Kollagen, Hydrokolloiden und/oder Farbstoffen bestehen, und wobei das Gitter oder Netz durch 3D-Printing, Siebdruck, Wirken, Weben, Sticken oder Stricken, erhältlich ist, als Primärauflage für die feuchte Wundversorgung.

## Claims

1. An elastic primary dressing for moist wound care, in the form of a mesh or netting produced from elongate elastic elements, wherein the mesh or netting contains non-resorbable elongate elements, wherein the non-resorbable elongate elements consist of silicone and at least one additive selected from pharmacologically active ingredients, collagen, hydrocolloids and/or colorants.

2. The primary dressing according to claim 1, wherein the mesh or netting additionally comprises resorbable elongate elements comprising at least one resorbable and bioactive organic compound selected from collagen, hydrocolloids and pharmacologically active ingredients.

3. The primary dressing according to claim 1 or 2, wherein the silicone is silicone that is to be cross-linked selected from liquid silicone, RTV silicone rubber, HTV silicone rubber and UV cross-linkable silicone.

4. The primary dressing according to any one of the preceding claims, wherein the surface of the mesh or netting has a resorbable coating which contains at least one pharmacologically active ingredient and/or at least one resorbable organic compound selected from collagen and hydrocolloids.

5. The primary dressing according to any one of the preceding claims, wherein antibacterially active ingredients and/or active ingredients for promoting wound healing are embedded in the silicone of the non-resorbable elongate elements and/or are present in a resorbable coating on the surface of the mesh or netting.

6. The primary dressing according to any one of the preceding claims, wherein the non-resorbable elongate elements form a pull tab disposed laterally on the mesh or netting.

7. A wound dressing for moist wound care, comprising:
- a primary dressing according to any one of claims 1 to 6, which is applied to the surface of a wound when in use, and
- a secondary dressing disposed on the side of the wound dressing which faces away from the wound surface when in use, wherein the secondary dressing contains a porous adsorbent element.

8. A method for the laminated manufacture of an elastic primary dressing for moist wound care in the form of a mesh formed from at least two layers of intersecting elongate elements wherein, in order to prepare the mesh, a first layer of mutually parallel elongate elements is applied and subsequently, a second layer of mutually parallel elongate elements is applied to the first layer, wherein the elongate elements of the first layer and the elongate elements of the second layer are disposed in a manner so as to be offset with respect to each other by an angle of 1° to 90° so that the elongate elements of the first and the second layers intersect, wherein the elongate elements are produced by extrusion from a viscous mass, wherein the mass consists of silicone to be cross-linked and pharmacologically active ingredients and/or colorants, and wherein after cross-linking of the silicone, an elastic solid can be obtained.

9. A method for manufacturing an elastic primary dressing for moist wound care in the form of a mesh, in which the mesh is produced by screen printing from a viscous mass, wherein the mass consists of silicone to be cross-linked and optional pharmacologically active ingredients and/or colorants and wherein the primary dressing is produced in the size employed for application.

10. The method according to claim 8 or 9, **characterized in that** the viscous mass formed from silicone to be cross-linked and pharmacologically active ingredients and/or colorants has a viscosity of 300,000 mPas to 1,000,000 mPas at a shear rate of 10 s⁻¹.

11. The method for the manufacture of an elastic primary dressing according to any one of claims 1 to 6 for moist wound care in the form of a mesh or netting, in which the mesh or netting is produced by weaving, warp knitting, bonding or weft knitting elongate elastic elements, wherein the elongate elastic elements consist of silicone and pharmacologically active ingredients and/or colorants.

12. The method according to any one of claims 9 to 11, wherein a pull tab formed from non-resorbable elongate elements is produced on a lateral end of the mesh or netting.

13. The method according to any one of claims 8 to 12, in which a resorbable coating which contains at least one pharmacologically active ingredient and/or at least one resorbable organic compound selected from collagen and hydrocolloids is applied to the surface of the mesh or netting.

14. Use of a mesh or netting which contains non-resorbable elongate elements, wherein the non-resorbable elongate elements consist of silicone and at least one additive selected from pharmacologically active ingredients, collagen, hydrocolloids and/or colorants, and wherein the mesh or netting can be obtained by 3D printing, screen printing, warp knitting, weaving, bonding or weft knitting, as a primary dressing for moist wound care.

## Revendications

1. Pansement primaire élastique pour le soin de plaies en milieu humide sous la forme d'une grille ou d'un filet à base de corps allongés élastiques, dans lequel la grille ou le filet contient des corps allongés non-résorbables, dans lequel les corps allongés non-résorbables se composent de silicone et d'au moins un additif sélectionné parmi des substances actives pharmacologiques, du collagène, des hydrocolloïdes et/ou des colorants.

2. Pansement primaire selon la revendication 1, dans lequel la grille ou le filet contient additionnellement des corps allongés résorbables comprenant au moins un composé organique résorbable et bioactif sélectionné parmi du collagène, des hydrocolloïdes et des substances actives pharmacologiques.

3. Pansement primaire selon la revendication 1 ou 2, dans lequel le silicone est du silicone réticulé sélectionné parmi du silicone liquide, du caoutchouc de silicone RTV, du caoutchouc de silicone HTV et du silicone réticulable aux UV.

4. Pansement primaire selon l'une des revendications précédentes, dans lequel il y a, sur la surface de la grille ou du filet, un revêtement résorbable qui contient au moins une substance active pharmacologique et/ou au moins un composé organique résorbable sélectionné parmi du collagène et des hydrocolloïdes.

5. Pansement primaire selon l'une des revendications précédentes, dans lequel des substances actives antibactériennes et/ou des substances actives pour favoriser la guérison de plaies sont noyées dans le silicone des corps allongés non-résorbables et/ou sont présents dans un revêtement résorbable à la surface de la grille ou du filet.

6. Pansement primaire selon l'une des revendications précédentes, dans lequel les corps allongés non-résorbables forment une languette à arracher disposée latéralement sur la grille ou le filet.

7. Pansement pour plaie, pour le soin de plaies en milieu humide, comprenant :
- un pansement primaire selon l'une des revendications 1 à 6 qui repose sur la surface de la plaie en cas d'utilisation, et
- un pansement secondaire qui est disposé sur le côté du pansement pour plaie, lequel, en cas d'utilisation, tourne le dos à la surface de la plaie, dans lequel le pansement secondaire contient un corps d'aspiration poreux.

8. Procédé pour la fabrication par couches d'un pansement primaire élastique pour le soin de plaies en milieu humide sous la forme d'une grille à base d'au moins deux couches de corps allongés se croisant, dans lequel, pour la mise à disposition de la grille, une première couche de corps allongés disposés parallèlement entre eux est appliquée et ensuite, une deuxième couche de corps allongés disposés parallèlement entre eux étant appliquée sur la première couche, dans lequel les corps allongés de la première couche et les corps allongés de la deuxième couche sont disposés de manière décalée entre eux d'un angle de 1 à 90° de sorte que les corps allongés des première et deuxième couches se croisent, dans lequel les corps allongés sont produits par extrusion à partir d'une masse visqueuse, dans lequel la masse se compose de silicone réticulé et de substances actives pharmacologiques et/ou de colorants, et dans lequel, après la réticulation du silicone, une matière solide élastique peut être obtenue.

9. Procédé pour la fabrication d'un pansement primaire élastique pour le soin de plaies en milieu humide sous la forme d'une grille, dans lequel la grille est produite par sérigraphie à partir d'une masse visqueuse, dans lequel la masse se compose de silicone réticulé et, le cas échéant, de substances actives pharmacologiques et/ou de colorants et dans lequel le pansement primaire est fabriqué aux dimensions d'utilisation.

10. Procédé selon l'une des revendications 8 ou 9, **caractérisé en ce que** la masse visqueuse à base de silicone réticulé et de substances actives pharmacologiques et/ou colorants présente une viscosité de 300 000 mPas à 1 000 000 mPas avec un taux de cisaillement de 10s⁻¹.

11. Procédé pour la fabrication d'un pansement primaire élastique selon l'une des revendications 1 à 6 pour le soin de plaies en milieu humide sous la forme d'une grille ou d'un filet, dans lequel la grille ou le filet est produit par tissage, tricotage, broderie, tricot de corps allongés élastiques, dans lequel les corps allongés élastiques se composent de silicone et de substances actives pharmacologiques et/ou de colorants.

12. Procédé selon l'une des revendications 9 à 11, dans lequel on produit, à une extrémité latérale de la grille ou du filet, une languette à arracher à base de corps allongés non-résorbables.

13. Procédé selon l'une des revendications 8 à 12, dans lequel un revêtement résorbable, lequel contient au moins une substance active pharmacologique et/ou au moins un composé organique résorbable sélectionné parmi du collagène et des hydrocolloïdes, est appliqué sur la surface de la grille ou du filet.

14. Utilisation d'une grille ou d'un filet qui contient des corps allongés non-résorbables, dans laquelle les corps allongés non-résorbables se composent de silicone et d'au moins un additif sélectionné parmi des substances actives pharmacologiques, du collagène, des hydrocolloïdes et/ou des colorants, et dans lequel la grille ou le filet peut être obtenu(e) par impression en 3D, sérigraphie, tricotage, tissage, broderie ou tricot, en tant que pansement primaire pour le soin de plaies en milieu humide.
